# EUROPEAN PATENT SPECIFICATION

(11) **EP 2 353 588 B1**
(45) Date of publication and mention of the grant of the patent: **15.04.2015**
(21) Application number: 10151282.0
(22) Date of filing: 21.01.2010
(51) Int. Cl.: A61K 9/16, A61K 38/48

(54) **A sustained release preparation of factor IX**
Präparat mit verzögerter Freisetzung von Faktor IX
Préparation à libération prolongée de facteur IX

(43) Date of publication of application: 10.08.2011
(73) Proprietor: Agricultural Technology Research Institute, Hsinchu City 300 (TW)
(72) Inventor: Chang, Li-Chien, 100, Taipei City (TW); Yang, Chi-Yu, Fengshan City (TW); Lin, Yi-Juain, Zhunan Township (TW)
(74) Representative: Viering, Jentschura & Partner Patent- und Rechtsanwälte

(56) References cited:
- EP-A2- 0 145 240
- WO-A1-94/28878
- US-A1- 2009 269 414
- DATABASE WPI Section Ch, Week 200950 Thomson Scientific, London, GB; Class A96, AN 2009-L06611 XP002581876 DOU X; GUO M; JIN T; WU F; YUAN W: "Preparation of microsphere comprises dissolving medicine in water, adding to slow-release or controlled-release organic solution, adding formed liquid suspension to another organic solution, emulsifying, solidifying, and freezing" -& CN 101 461 785 A (UNIV SHANGHAI JIAOTONG) 24 June 2009 (2009-06-24)
- DATABASE WPI Section Ch, Week 200950 Thomson Scientific, London, GB; Class A96, AN 2009-L06610 XP002581877 JIN T; REN T; WU F; YUAN W; ZHAO H: "New polylactic acid (PLA)/copolylactic acid/glycolic acid (PLGA) shell-core microspheres, used in medical applications" -& CN 101 461 786 A (UNIV SHANGHAI JIAOTONG) 24 June 2009 (2009-06-24)

## Description

### FIELD OF THE INVENTION

This invention relates to a sustained preparation encapsulated Factor IX.

### BACKGROUND OF THE INVENTION

Factor IX is one of the serine proteases of the coagulation system in the body. Deficiency of such protein causes Hemophilia B, a blood clotting disorder, which is also known as Christmas disease. Patients with hemophilia B are more prone to bleeding than normal and have poor wound healing after injury or surgery. Bleeding can happen within muscles or the spaces in the joints, such as the elbows, knees and ankles. Rare, but life-threatening bleeding can also happen in the brain and spinal cord, the throat or the gut. Hemophilia B is a lifelong debilitating disease that is life threatening.

Generally, the conventional treatment of hemophilia B patients is intravenous injection of recombinant factor IX, i.e. commercially available BeneFIX^{®} (Wyeth), to replace the missing or mutated protein. However, therapeutic polypeptide drugs are rapidly degraded by proteolytic enzymes or neutralized by antibodies. This reduces their half-life and circulation time, thereby limiting their therapeutic effectiveness.

PEGylation of proteins is a well-established technique in protein chemistry and plays an important role in current drug delivery to enhance protection from proteolytic degradation. For example, PCT International Application WO 2009/083187 A1 provided a chemically modified blood coagulation factor IX (Factor IX) comprising a Factor IX activation peptide region (AP region), wherein said AP region comprises a covalently coupled water-soluble hydrophilic polymer. In one preferred embodiment of the PCT application, the PEG was attached to Factor IX via Asn-157 and/or Asn-167 of Factor IX. In an alternatively preferred embodiment, the PEG was attached to FIX via Ser-158, Thr-159, Thr-163, Thr-169, Ser-171 , Thr-172, Ser-174 or Thr-179, especially via Ser-158, Thr-163, Ser-171 or Ser-174 of Factor IX, especially if the water- soluble hydrophilic polymer is PEG. However, such addition of PEG would inevitably change the structure of the polypeptide and might cause unexpected side effects.

CN 101 461 785 A also discloses a preparation comprising Factor IX, more specifically a slow-release or controlled-release preparation comprising microspheres comprising Factor IX and poly(lactic-co-glycolic acid), said preparation being manufactured by a water-in-oil-in-oil-in-water emulsion method.

### SUMMARY OF THE INVENTION

The present invention features a new pharmaceutical preparation in a powder-like form comprising human Factor IX encapsulated by a lipophilic biodegradable polymer or copolymer to form a microsphere, which provides a prolonged release of human Factor IX, and is free of any remnant organic solution after lyophilization.

In one aspect, the present invention provides a pharmaceutical preparation in a powder-like form according to claim 1 comprising a therapeutically effective amount of human Factor IX (hFIX), which is encapsulated by a lipophilic biodegradable polymer or copolymer to form a microsphere, whereby the pharmaceutical preparation provides a sustained release of hFIX and a prolonged biological activity, wherein the lipophilic biodegradable polymer or copolymer is selected from the group consisting of polyglycolic acid (PGA), poly(lactic-co-glycolic acid) (PLGA), poly(γ-glutamic acid), polyvinylic acid (PVA), polycaprolactone, polyanhydrides, polyamino acid, polydioxanone, polyhydroxybutyrate, polyphosphazenes, polyesterurethane, polycarboxyphenoxypropane-cosebacic acid, polyorthoester, and the combination thereof.

In the other aspect, the present invention provides a method according to claim 6 for manufacturing the pharmaceutical preparation of the present invention consisting of the following steps
a) mixing a therapeutically effective amount of human Factor IX (hFIX) in an aqueous solution with a lipophilic biodegradable polymer or copolymer in an organic solution to obtain a primary emulsion;
b) mixing the primary emulsion with a surfactant solution to obtain a secondary emulsion; and
c) evaporating the organic solvent, then filtrating, washing, and lyophilizing the secondary emulsion to obtain a powder-like pharmaceutical preparation;
whereby in the power-like pharmaceutical preparation, hFIX is encapsulated by the lipophilic biodegradable polymer or copolymer to form a microsphere so as to provide a sustained release of hFIX and a prolonged biological activity.

The details of one or more embodiments of the invention are set forth in the description below. Other features or advantages of the present invention will be apparent from the following drawings and detailed description of several embodiments, and also from the appending claims.

### BRIEF DESCRIPTION OF THE DRAWINGS

The above aspects and advantages of the present invention will become more readily apparent to those ordinarily skilled in the art after reviewing the following detailed description and accompanying drawings, in which:

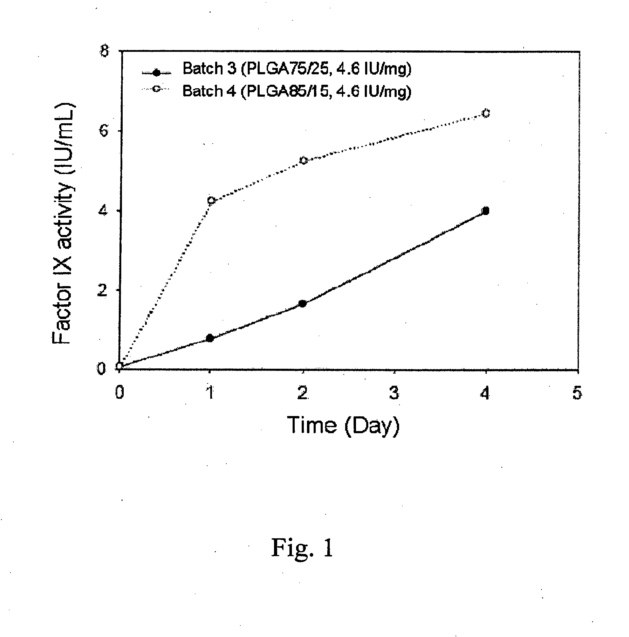

Fig. 1 is a diagram showing the hFIX activities of the pharmaceutical preparation according to the present invention, which were determined by the aPTT assay at different times for the preparations of Batch 3 (PLGA75/25, 4.6 IU/mg) and Batch 4 (PLGA85/15, 4.6 IU/mg).

### DETAILED DESCRIPTION OF THE INVENTION

Unless defined otherwise, all technical and scientific terms used herein have the meaning commonly understood by a person skilled in the art to which this invention belongs.

The articles "a" and "an" are used herein to refer to one or more than one (*i.e*., at least one) of the grammatical object of the article.

The invention provides a pharmaceutical preparation in a powder-like form according to claim 1 comprising a therapeutically effective amount of human Factor IX (hFIX), which is encapsulated by a lipophilic biodegradable polymer or copolymer to form a microsphere, whereby the pharmaceutical preparation provides a sustained release of hFIX and a prolonged biological activity. The hFIX may be naturally occurring or recombinant Factor IX. According to the invention, the pharmaceutical preparation is used for treating hemophilia B. The term "treating" used herein refers to curing, ameliorating or meliorating the blood bleeding disorder in the subject.

The term "microsphere" used herein refers to a small spherical particle, with diameters in the micrometer range. In one embodiment of the invention, the microsphere has a diameter of 0.1 to 100 µm. The microspheres of the present invention are further lyophilized to be in a powder-like form for easy transportation, manipulation, and administration.

The term "biodegradable polymer or copolymer" herein refers to the polymer or copolymer, which is degraded or eroded *in vivo,* for example, by enzymatic, chemical and/or physical processes, to form smaller chemical species.

The term "lipophilic" used herein refers to the biodegradable polymer or copolymer to dissolve in fats, oils, lipids, and organic solvents. In the invention, the lipophilic biodegradable polymer or copolymer is selected from a group consisting of polyglycolic acid (PGA), poly(lactic-co-glycolic acid) (PLGA), poly(γ-glutamic acid), polyvinylic acid (PVA), polycaprolactone, polyanhydrides, polyamino acid, polydioxanone, polyhydroxybutyrate, polyphosphazenes, polyesterurethane, polycarboxyphenoxypropane-cosebacic acid, polyorthoester, and the combination thereof. In one example of the invention, the lipophilic biodegradable polymer or copolymer is PLGA, for example in a concentration of approximately 1 mg/ml to 1000 mg/ml, preferably 90 mg/ml.

Depending on the ratio of lactide to glycolide used for the polymerization, different forms of PLGA can be obtained: these are usually identified in regard to the monomers' ratio used (e.g. PLGA 75:25 identifies a copolymer whose composition is 75% lactic acid and 25% glycolic acid). According to the invention, the monomers' ratio is PLGA 10/90 to PLGA 90/10. In one embodiment, PLGA 75/25 or PLGA 85/15 is used for manufacturing the pharmaceutical preparation of the invention.

Typically, the loading efficiency, or the encapsulation ratio of hFIX in the microspheres is at least 80% by total mass. The loading dosage varies in density for different purposes and is dependent on the dosing regimen which is commonly based on the patients' individual pharmacokinetics. The encapsulation ratio may be adjusted by anyone skilled in the art according to standard technologies.

The term "an effective amount" refers to the amount that is required to confer the intended therapeutic effect in the subject to be administrated. Effective amounts may vary, as recognized by those skilled in the art, depending on routes of administration, excipient usages, and the possibilities of co-usage with other agents. Preferably, the pharmaceutical preparation can be administered intravenously, intramuscularly or subcutaneously.

The pharmaceutical preparation may further comprise one or more pharmaceutically acceptable carriers, which may be dissolved in an aqueous solution. Such carriers may include, but not limit to: saline, buffered saline, dextrose, water, glycerol, ethanol and combinations thereof.

The present invention also provided a method according to claim 6 for manufacturing the pharmaceutical preparation of the present invention consisting of the following steps:
a) mixing a therapeutically effective amount of human Factor IX (hFIX) in an aqueous solution with a lipophilic biodegradable polymer or copolymer in an organic solution to obtain a primary emulsion;
b) mixing the primary emulsion with a surfactant solution to obtain a secondary emulsion; and
c) evaporating the organic solvent, then filtrating, washing, and lyophilizing the secondary emulsion to obtain a powder-like pharmaceutical preparation;
whereby in the powder-like pharmaceutical preparation, hFIX is encapsulated by the lipophilic biodegradable polymer or copolymer to form a microsphere so as to provide a sustained release of hFIX and a prolonged biological activity.

According to the invention, the microsphere is made in the form of water-in-oil-in-water (W/O/W) emulsion. The hFIX is dissolved in the first aqueous solution at the concentration of 0.001 % to 90% by mass according to the first aqueous solution. In one embodiment of the invention, the amount of hFIX is 20 mg. The first aqueous solution containing hFIX is then mixed with an organic solution which contains at least one lipophilic biodegradable polymer or copolymer in a ratio of 1:1 to 1:100 (v:v) to obtain the primary emulsion. In one embodiment of the invention, the aqueous solution and the organic solution are in a ratio of 1:10.

According to the invention, the organic solvent to form an organic solution is selected from a group consisting of dichloromethane, chloroform, ethyl acetate, 1,4-dioxane, dimethyl formamide (DMF), dimethyl sulfoxide (DMSO), toluene, and tetrahydrofuran (THF). In one embodiment of the invention, the organic solvent is dichloromethane.

Optionally, the solution used to make the primary emulsion may further contain at least one surfactant to facilitate the production of the primary emulsion. In one embodiment, the one or more surfactant is selected from a group consisting of glycerin fatty acid ester, sucrose fatty acid ester, sorbitan fatty acid ester, propylene glycol fatty acid ester, diacetyl tartaric acid esters of mono-and diglycerides, sodium aluminum phosphate, polysorbate 20, polysorbate 60, polysorbate 65, polysorbate 80, hydroxypropyl cellulose, hydroxylpropyl methylcellulose, citrated mono-and diglycerides tartrated, mono-and diglycerides lactated, mono-and diglycerides ethoxylated, mono-and diglycerides, mono-and diglycerides monosodium phosphate derivatives, succinylated monoglycerides, polyglycerol esters of fatty acid, polyglycerol esters of interesterified ricinoleic acids, calcium stearyl-2-lactylate, salts of fatty acids, polyoxyethylene(20) sorbitan monopalmitate, polysorbate 40, polyoxyethylene(20) monostearate, polyoxyethylene(20) sorbitan tristearate, Triton x-100, polyethylene glycol 200-800, sodium lauryl sulfate, alcohol ethoxylates, alkylphenol ethoxylates, alkyl polyglycosides, and the combination thereof.

According to the invention, the primary emulsion is mixed with a surfactant solution to obtain a secondary emulsion. The surfactant is selected from the group consisting of glycerin fatty acid ester, sucrose fatty acid ester, sorbitan fatty acid ester, propylene glycol fatty acid ester, diacetyl tartaric acid esters of mono-and diglycerides, sodium aluminum phosphate, polysorbate 20, polysorbate 60, polysorbate 65, polysorbate 80, hydroxypropyl cellulose, hydroxylpropyl methylcellulose, citrated mono-and diglycerides tartrated, mono-and diglycerides lactated, mono-and diglycerides ethoxylated, mono-and diglycerides mono-and diglycerides monosodium phosphate derivatives, succinylated monoglycerides, polyglycerol esters of fatty acid, polyglycerol esters of interesterified ricinoleic acids, calcium stearyl-2-lactylate, salts of fatty acids, polyoxyethylene(20) sorbitan monopalmitate, polysorbate 40, polyoxyethylene(20) monostearate, polyoxyethylene(20) sorbitan tristearate, Triton x-100, polyethylene glycol 200-800, sodium lauryl sulfate, alcohol ethoxylates, alkylphenol ethoxylates, alkyl polyglycosides, or the combination thereof. In one embodiment of the invention, the surfactant is PVA, such as at a concentration of approximately 0.1 % to 1.0% in the second aqueous solution, preferably 0.5% PVA.

According to the invention, the ratio of the surfactant solution and the primary emulsion may be 1:1 to 1000:1 to obtain the secondary emulsion. In one example of the invention, the ratio is 30:1. The secondary emulsion after evaporation of the organic solvent is subsequently filtered, washed and lyophilized by conventional methods in the art to obtain a powder-like pharmaceutical preparation. The evaporation time can be set from 0.1 to 24 hours. In one embodiment of the invention, the evaporation time is preferably for 3 to 4 hours.

To disperse two or more immiscible liquids, such as the emulsification of oil into water, ultrasonication may be carried out through any known or commonly used methods or technologies. According to the invention, the power for ultrasonication may be 10 to 500 W for 0.01 to 30 min. In one preferable embodiment of the invention, the ultrasonic power is 75W for 2-3 min. Any skilled person in the art may choose the suitable condition according to different loaded dosages or different materials used through repeated experiments.

The present invention is further illustrated by the following examples, which are provided for the purpose of demonstration rather than limitation.

Example 1: Preparation of the Prolonged Release Microsphere Encapsulated Human Recombinant Factor IX.

Recombinant human factor IX (rhFIX) was obtained from transgenic pig milk which was provided from Animal Technology Institute, Taiwan. After collecting the milk, the pig milk was centrifuged by 3,000 x *g*, at 4°C for 20 minutes to remove fat. The fraction containing rhFIX as obtained was prepared from skimmed milk by precipitation with phosphate buffer solution and centrifugation by 22860 x g, at 4°C for 10 minutes. The whey fraction was further concentrated by ultrafiltration using a polysulfone membrane with a molecular cutoff of 30 kD (TAMI). The rhFIX was subsequently captured and purified by Q Sepharose fast flow chromatography (Amersham Pharmacia Biotech) and Heparin-Sepharose column (Amersham Pharmacia Biotech). Nanofiltration was used to be the viral removal step.

The rhFIX dissolved in a distilled water and 90 mg/ml polylacto-glycolic acid (PLGA) dissolved in dichloromethane in a ratio of 1:10 (v:v) were mixed, and then the mixture was ultrasonicated at 75 W for 2-3 min to obtain a primary emulsion. The primary emulsion was further mixed with 0.5% PVA in a ratio of 1:30 (v:v) and ultrasonicated at 75 W for 2-3 min to obtain a secondary emulsion. After the organic solvent was volatilized from the secondary emulsion for approximately 3-4 hours, the secondary emulsion was filtrated, washed and lyophilized to obtain PLGA microsphere encapsulated rhFIX. The filtered solution and cleaning liquid was collected during the procession, and combined as "combined solution" which was used to determine the encapsulated ratio and drug dosage.

Example 2: Determining Release Kinetics and Activity of encapsulated rhFIX by an *in vitro* Simulated physiological Test

The simulated physiological test was performed by adding the prolonged release microsphere produced from Example 1 into a physiological buffer (0.5% Tween 20, PBS, pH7.4) maintained at 37°C . Small aliquots of the buffer were collected to determine the activity of rhFIX released from the microsphere every day. The rhFIX activity was determined by an aPTT (Activated Partial Thromboplastin Time) test while using FIX deficient plasma as the control.

The aPTT test is a clotting time test which is used for determining FIX activity in a sample. Briefly, a fixed amount (25 µl) of plasma sample was mixed 50 µl aPTT reagent and stand at 37°C for 1 min. Then, 50 µl 0.025 M CaCl₂ was added into the sample to initiate the analysis, which is performed at 37°C for 4 min, under 660 nm by an automated blood clot coagulation analyzer (TECO Coatron M4), whose principle is to measure the change of light scattered from the sample as it clots. The results of the in vitro test were as shown in Fig 1.

As shown in Fig. 1, the rhFIX released from the preparations according to the invention were tested in vitro and found that the activities were remained and gradually released from the preparations of PLGA85/15 (Batch 4) and PLGA 75/25 (Batch 3) for four days.

Example 3: An *in vivo* Test on the Bioactivity of the rhFIX Released From the Microsphere.

A hemophilia B mice model was used to determine the prolonged release of rhFIX from the preparation according to the invention, and non-encapsulated rhFIX as a comparison. Six hemophilia B mice (R333Q-hFIX mice are gifts from Darrel W. Stafford, Department of Biology, UNC Chapel Hill, Chapel Hill) for each group were given 50 IU/kg rhFIX to test. Non-encapsulated rhFIX and the preparation according to the invention were administered intravenously through the tail vein and subcutaneously at the abdominal site, respectively. At different times after the administration, blood was collected from each mouse and centrifuged to obtain the plasma sample. The activity was determined by the aPTT assay as mentioned above. Blood clotting time which exceeds 120 seconds was deemed as weak or no activity of rhFIX. The detailed data was summarized in Table I. Data are presented as mean ± standard deviation unless otherwise noted. Statistical evaluation was performed with independent *t* test. (SPSS version 12.0, Claritas Inc.). Value of p<0.05 was considered statistically significant.

As shown in Table I, the ability of rhFIX on shortening blood coagulation time lasted for 48 hours (2 days) and became insignificant 72 hours (3days) after the administration in the group treated with the non-encapsulated rhFIX. On the contrary, in the group treated with the preparation according to the invention, a prolonged bioactivity of rhFIX was observed for at least 120 hours (5 days), and became insignificant until 168 hours (7 days) after the administration.

**Table I**

| | Blood Clotting Time (aPTT, sec) | |
|---|---|---|
| Sampling Time | Encapsulated rhFIX | Non-encapsulated rhFIX |
| 0.25 hr | 37.40±5.41 | 42.82±2.74 (*p*=0.078) |
| 24 hr | 41.87±14.74 | 74.58±8.29 (*p*< 0.01) |
| 48 hr | 51.40±7.39 | 81.33±3.45 (*p*< 0.01) |
| 72 hr | 58.63±3.66 | 121.17±16.93 (*p*< 0.01) |
| 96 hr | 68.37±2.90 | >120 sec |
| 120 hr | 63.70±5.74 | >120 sec |
| 168 hr | 92.73±5.31 | - |

Given the above, it was concluded that the pharmaceutical preparation of the present invention provided a prolonged release of rhFIX, and the bioactivity of rhFIX *in vivo* maintained for at least 5 days. Therefore, the frequency of the injection may be decreased to 1 every 5 or 7 days, and it would significantly reduce the hemophilia B patient's burden. Furthermore, the preparation in a powder-like form generally provides a better chemical stability and could be stored for a longer period.

## Claims

1. A pharmaceutical preparation in a powder-like form comprising a therapeutically effective amount of human Factor IX (hFIX), which is encapsulated by a lipophilic biodegradable polymer or copolymer to form a microsphere being made from a water-in-oil-in-water (w/o/w) emulsion, whereby the pharmaceutical preparation provides a sustained release of hFIX and a prolonged biological activity, the pharmaceutical preparation being prepared by a method consisting of the following steps:
a) mixing a therapeutically effective amount of human Factor IX (hFIX) in an aqueous solution with a lipophilic biodegradable polymer or copolymer in an organic solution to obtain a primary emulsion;
b) mixing the primary emulsion with a surfactant solution to obtain a secondary emulsion; and
c) evaporating the organic solvent, then filtrating, washing, and lyophilizing the secondary emulsion to obtain a powder-like pharmaceutical preparation;
wherein the lipophilic biodegradable polymer or copolymer is selected from the group consisting of polyglycolic acid (PGA), poly(lactic-co-glycolic acid) (PLGA), poly(γ-glutamic acid), polyvinylic acid (PVA), polycaprolactone, polyanhydrides, polyamino acid, polydioxanone, polyhydroxybutyrate, polyphosphazenes, polyesterurethane, polycarboxyphenoxypropane-cosebacic acid, polyorthoester, and the combination thereof, and wherein the the surfactant is selected from a group consisting of glycerin fatty acid ester, sucrose fatty acid ester, sorbitan fatty acid ester, propylene glycol fatty acid ester, diacetyl tartaric acid esters of mono-and diglycerides, sodium aluminum phosphate, polysorbate 20, polysorbate 60, polysorbate 65, polysorbate 80, hydroxypropyl cellulose, hydroxylpropyl methylcellulose, citrated mono-and diglycerides, tartrated mono-and diglycerides, lactated mono-and diglycerides, ethoxylated mono-and diglycerides, mono-and diglycerides monosodium phosphate derivatives, succinylated monoglycerides, polyglycerol esters of fatty acid, polyglycerol esters of interesterified ricinoleic acids, calcium stearyl-2-lactylate, salts of fatty acids, polyoxyethylene(20) sorbitan monopalmitate, polysorbate 40, polyoxyethylene(20) monostearate, polyoxyethylene(20) sorbitan tristearate, Triton x-100, polyethylene glycol 200-800, sodium lauryl sulfate, alcohol ethoxylates, alkylphenol ethoxylates, alkyl polyglycosides, and combination thereof.

2. The pharmaceutical preparation of claim 1 for use in treating hemophilia B.

3. The pharmaceutical preparation of claim 1, wherein the lipophilic biodegradable polymer or copolymer is polylacto-glycolic acid (PLGA).

4. The pharmaceutical preparation of claim 1, wherein the microsphere has a diameter from 0.1 to 100 µm.

5. The pharmaceutical preparation of claim 1, wherein hFIX is in an amount of 0.0005 U/mg to 25 U/mg.

6. A method for manufacturing a pharmaceutical preparation according to claim 1 consisting of the following steps:
a) mixing a therapeutically effective amount of human Factor IX (hFIX) in an aqueous solution with a lipophilic biodegradable polymer or copolymer in an organic solution to obtain a primary emulsion;
b) mixing the primary emulsion with a surfactant solution to obtain a secondary emulsion; and
c) evaporating the organic solvent, then filtrating, washing, and lyophilizing the secondary emulsion to obtain a powder-like pharmaceutical preparation;
whereby in the powder-like pharmaceutical preparation, hFIX is encapsulated by the lipophilic biodegradable polymer or copolymer to form a microsphere so as to provide a sustained release of hFIX and a prolonged biological activity.

7. The method of claim 6, wherein the solution of step (a) may further contain a surfactant.

8. The method of claim 6, wherein the lipophilic biodegradable polymer or copolymer is selected from a group consisting of polyglycolic acid (PGA), poly(lactic-co-glycolic acid) (PLGA), poly(γ-glutamic acid), polyvinylic acid (PVA), polycaprolactone, polyanhydrides, polyamino acid, polydioxanone, polyhydroxybutyrate, polyphosphazenes, polyesterurethane, polycarboxyphenoxypropane-cosebacic acid, polyorthoester, and combination thereof.

9. The method of claim 6, wherein the lipophilic biodegradable polymer or copolymer is polylacto-glycolic acid (PLGA).

10. The method of claim 6, wherein the surfactant is selected from a group consisting of glycerin fatty acid ester, sucrose fatty acid ester, sorbitan fatty acid ester, propylene glycol fatty acid ester, diacetyl tartaric acid esters of mono-and diglycerides, sodium aluminum phosphate, polysorbate 20, polysorbate 60, polysorbate 65, polysorbate 80, hydroxypropyl cellulose, hydroxylpropyl methylcellulose, citrated mono-and diglycerides, tartrated mono- and diglycerides, lactated mono-and diglycerides, ethoxylated mono-and diglycerides, mono- and diglycerides monosodium phosphate derivatives, succinylated monoglycerides, polyglycerol esters of fatty acid, polyglycerol esters of interesterified ricinoleic acids, calcium stearyl-2-lactylate, salts of fatty acids, polyoxyethylene(20) sorbitan monopalmitate, polysorbate 40, polyoxyethylene(20) monostearate, polyoxyethylene(20) sorbitan tristearate, Triton x-100, polyethylene glycol 200-800, sodium lauryl sulfate, alcohol ethoxylates, alkylphenol ethoxylates, alkyl polyglycosides, and combination thereof.

11. The method of claim 6, wherein the organic solvent is selected from a group consisting of dichloromethane, chloroform, ethyl acetate, 1,4-dioxane, dimethyl formamide (DMF), dimethyl sulfoxide (DMSO), toluene, tetrahydrofuran (THF), and combination thereof.

## Patentansprüche

1. Eine pharmazeutische Zubereitung in einer pulverartigen Form umfassend eine therapeutisch wirksame Menge von humanem Faktor IX (hFIX), der durch ein lipophiles, biologisch abbaubares Polymer oder Copolymer eingekapselt ist, um so eine Mikrosphäre, die aus einer Wasser-in-Öl-in-Wasser- (w/o/w) Emulsion besteht, zu bilden, wobei die pharmazeutische Zubereitung eine anhaltende Freisetzung von hFIX und eine verlängerte biologische Aktivität sicherstellt, wobei die pharmazeutische Zubereitung mittels einer Methode bestehend aus den folgenden Schritten hergestellt wird:
a) Mischen einer therapeutisch wirksamen Menge eines humanen Faktors IX (hFIX) in einer wässrigen Lösung mit einem lipophilen, biologisch abbaubaren Polymer oder Copolymer in einer organischen Lösung, um eine erste Emulsion zu erhalten;
b) Mischen der ersten Emulsion mit einer Tensidlösung, um eine zweite Emulsion zu erhalten; und
c) Verdampfen des organischen Lösemittels, dann Filtrieren, Waschen und Lyophilisieren der zweiten Emulsion, um eine pulverartige pharmazeutische Zubereitung zu erhalten;
wobei das lipophile, biologisch abbaubare Polymer oder Copolymer ausgewählt ist aus der Gruppe bestehend aus Polyglycolsäure (PGA), Poly(Milchsäure-co-Glycolsäure) (PLGA), Poly(γ-Glutaminsäure), Polyvinylsäure (PVA), Polycaprolacton, Polyanhydriden, Polyaminosäure, Polydioxanon, Polyhydroxybutyrat, Polyphosphazenen, Polyesterurethan, Polycarboxy-phenoxypropan-Cosebacinsäure, Polyorthoester und Kombinationen davon, und wobei das Tensid ausgewählt ist aus der Gruppe bestehend aus Glycerinfettsäureester, Saccharosefettsäureester, Sorbitanfettsäureester, Propylen-glycolfettsäureester, Diacetylweinsäureestern von Mono- und Diglyceriden, Natriumaluminiumphosphat, Polysorbat 20, Polysorbat 60, Polysorbat 65, Polysorbat 80, Hydroxypropylcellulose, Hydroxypropylmethylcellulose, Citronensäure-veresterte Mono- und Diglyceride, Weinsäure-veresterte Mono- und Diglyceride, Milchsäure-veresterte Mono- und Diglyceride, ethoxylierte Mono- und Diglyceride, Mononatriumphosphat-Derivate von Mono- und Digylceriden, Bernsteinsäure-veresterte Monoglyceride, Polyglycerolester von Fettsäuren, Polyglycerolester von zwischenveresterten Ricinolsäuren, Calciumstearyl-2-lactylat, Salzen von Fettsäuren, Polyoxyethylen(20)sorbitan-monopalmitat, Polysorbat 40, Polyoxyethylen(20)monostearat, Polyoxyethylen(20)sorbitantristearat, Triton x-100, Polyethylenglycol 200-800, Natriumlaurylsulfat, Alkoholethoxylaten, Alkylphenolethoxylaten, Alkylpolyglycosiden und Kombinationen davon.

2. Die pharmazeutische Zubereitung gemäß Anspruch 1 zur Verwendung in der Behandlung von Hämophilie B.

3. Die pharmazeutische Zubereitung gemäß Anspruch 1, wobei das lipophile, biologisch abbaubare Polymer oder Copolymer Polylactoglycolsäure (PLGA) ist.

4. Die pharmazeutische Zubereitung gemäß Anspruch 1, wobei die Mikrosphäre einen Durchmesser von 0,1 bis 100 µm hat.

5. Die pharmazeutische Zubereitung gemäß Anspruch 1, wobei hFIX in einer Menge von 0,0005 U/mg bis 25 U/mg vorliegt.

6. Ein Verfahren zur Herstellung einer pharmazeutischen Zubereitung gemäß Anspruch 1,
bestehend aus den folgenden Schritten:
a) Mischen einer therapeutisch wirksamen Menge von humanem Faktor IX (hFIX) in einer wässrigen Lösung mit einem lipophilen, biologisch abbaubaren Polymer oder Copolymer in einer organischen Lösung, um eine erste Emulsion zu erhalten;
b) Mischen der ersten Emulsion mit einer Tensidlösung, um eine zweite Emulsion zu erhalten; und
c) Verdampfen des organischen Lösemittels, dann Filtrieren, Waschen und Lyophilisieren der zweiten Emulsion, um eine pulverartige pharmazeutische Zubereitung zu erhalten;
wobei, in der pulverartigen pharmazeutischen Zubereitung, hFIX durch ein lipophiles, biologisch abbaubares Polymer oder Copolymer eingekapselt ist, um so eine Mikrosphäre zu bilden, so dass eine anhaltende Freisetzung des hFIX und eine verlängerte biologische Aktivität gegeben ist.

7. Das Verfahren gemäß Anspruch 6, wobei die Lösung aus Schritt a) des Weiteren ein Tensid enthalten kann.

8. Das Verfahren gemäß Anspruch 6, wobei das lipophile, biologisch abbaubare Polymer oder Copolymer ausgewählt ist aus der Gruppe bestehend aus Polyglycolsäure (PGA), Poly(Milchsäure-co-Glycolsäure) (PLGA), Poly(y-Glutaminsäure), Polyvinylsäure (PVA), Polycaprolacton, Polyanhydriden, Polyaminosäure, Polydioxanon, Polyhydroxybutyrat, Polyphosphazenen, Polyesterurethan, Polycarboxyphenoxypropan-Cosebacinsäure, Polyorthoester und Kombinationen davon.

9. Das Verfahren gemäß Anspruch 6, wobei das lipophile, biologisch abbaubare Polymer oder Copolymer Polylactoglycolsäure (PLGA) ist.

10. Das Verfahren gemäß Anspruch 6, wobei das Tensid ausgewählt ist aus der Gruppe bestehend aus Glycerinfettsäureester, Saccharosefettsäureester, Sorbitanfettsäureester, Propylenglycolfettsäureester, Diacetylweinsäureestern von Mono- und Diglyceriden, Natriumaluminiumphosphat, Polysorbat 20, Polysorbat 60, Polysorbat 65, Polysorbat 80, Hydroxypropylcellulose, Hydroxypropylmethylcellulose, Citronensäure-veresterte Mono- und Diglyceride, Weinsäure-veresterte Mono- und Diglyceride, Milchsäure-veresterte Mono- und Diglyceride, ethoxylierte Mono- und Diglyceride, Mononatriumphosphat-Derivate von Mono- und Digylceriden, Bernsteinsäure-veresterte Monoglyceride, Polyglycerolester von Fettsäuren, Polyglycerolester von zwischenveresterten Ricinolsäuren, Calciumstearyl-2-lactylat, Salzen von Fettsäuren, Polyoxyethylen(20)-sorbitanmonopalmitat, Polysorbat 40, Polyoxyethylen(20)monostearat, Polyoxyethylen(20)sorbitantristearat, Triton x-100, Polyethylenglycol 200-800, Natriumlaurylsulfat, Alkoholethoxylaten, Alkylphenolethoxylaten, Alkylpolyglycosiden und Kombinationen davon.

11. Das Verfahren gemäß Anspruch 6, wobei das organische Lösemittel ausgewählt ist aus der Gruppe bestehend aus Dichlormethan, Chloroform, Ethylacetat, 1,4-Dioxan, Dimethylformamid (DMF), Dimethylsulfoxid (DMSO), Toluol, Tetrahydrofuran (THF) und Kombinationen davon.

## Revendications

1. Préparation pharmaceutique sous forme de poudre comprenant une quantité efficace du point de vue thérapeutique de facteur IX humain (hFIX) qui est encapsulé par un polymère ou copolymère biodégradable lipophile pour former une microsphère faite à partir d'une émulsion eau dans l'huile dans l'eau (w/o/w), la préparation pharmaceutique assurant une libération prolongée de hFIX et une activité biologique prolongée, la préparation pharmaceutique étant préparée par un procédé consistant en les étapes suivantes :
a) mélange d'une quantité efficace du point de vue thérapeutique de facteur IX humain (hFIX) dans une solution aqueuse avec un polymère ou copolymère biodégradable lipophile dans une solution organique pour former une émulsion primaire ;
b) mélange de l'émulsion primaire avec une solution de tensioactif pour former une émulsion secondaire ; et
c) évaporation du solvant organique, puis filtration, lavage et lyophilisation de l'émulsion secondaire pour former une préparation pharmaceutique en forme de poudre ;
dans laquelle le polymère ou copolymère biodégradable lipophile est choisi dans l'ensemble constitué par le poly(acide glycolique) (PGA), le copoly(acide lactique/acide glycolique) (PLGA), le poly(acide γ-glutamique), le poly(acide vinylique) (PVA), la polycaprolactone, les polyanhydrides, un poly(acide aminé), la polydioxanone, un polyhydroxybutyrate, les polyphosphazènes, un polyester-uréthane, le copoly(carboxyphénoxypropane/acide sébacique), un polyorthoester, et leurs combinaisons, et dans laquelle le tensioactif est choisi dans l'ensemble constitué par un ester d'acide gras et de glycérol, un ester d'acide gras et de saccharose, un ester d'acide gras et de sorbitan, un ester d'acide gras et de propylèneglycol, les esters d'acide diacétyltartrique et de mono- et di-glycérides, le phosphate de sodium-aluminium, le polysorbate 20, le polysorbate 60, le polysorbate 65, le polysorbate 80, l'hydroxypropylcellulose, l'hydroxypropylméthyl-cellulose, les mono- et di-glycérides citratés, les mono- et di-glycérides tartratés, les mono- et di-glycérides lactatés, les mono- et di-glycérides éthoxylés, les dérivés mono- et di-glycéridiques de phosphate monosodique, les monoglycérides succinylatés, les esters de polyglycérol et d'acide gras, les esters de polyglycérol et d'acide ricinoléique inter-estérifié, le stéaryl-2-lactylate de calcium, les sels d'acides gras, le monopalmitate de sorbitan polyoxyéthyléné(20), le polysorbate 40, un monostéarate polyoxyéthyléné(20), le tristéarate de sorbitan polyoxyéthyléné(20), le Triton X-100, les polyéthylèneglycols 200-800, le laurylsulfate de sodium, les alcools éthoxylés, les alkylphénols éthoxylés, les alkylpolyglycosides, et leurs combinaisons.

2. Préparation pharmaceutique selon la revendication 1 pour utilisation dans le traitement de l'hémophilie B.

3. Préparation pharmaceutique selon la revendication 1, dans laquelle le polymère ou copolymère biodégradable lipophile est le copoly(acide lactique/acide glycolique) (PLGA).

4. Préparation pharmaceutique selon la revendication 1, dans laquelle la microsphère a un diamètre de 0,1 à 100 µm.

5. Préparation pharmaceutique selon la revendication 1, dans laquelle hFIX est présent en une quantité de 0,0005 U/mg à 25 U/mg.

6. Procédé pour fabriquer une préparation pharmaceutique selon la revendication 1,
consistant en les étapes suivantes :
a) mélange d'une quantité efficace du point de vue thérapeutique de facteur IX humain (hFIX) dans une solution aqueuse avec un polymère ou copolymère biodégradable lipophile dans une solution organique pour former une émulsion primaire ;
b) mélange de l'émulsion primaire avec une solution de tensioactif pour former une émulsion secondaire ; et
c) évaporation du solvant organique, puis filtration, lavage et lyophilisation de l'émulsion secondaire pour former une préparation pharmaceutique en forme de poudre ;
de façon que, dans la préparation pharmaceutique en forme de poudre, hFIX soit encapsulé par le polymère ou copolymère biodégradable lipophile pour former une microsphère de façon à permettre une libération prolongée de hFIX et une activité biologique prolongée.

7. Procédé selon la revendication 6, dans lequel la solution de l'étape (a) peut en outre contenir un tensioactif.

8. Procédé selon la revendication 6, dans lequel le polymère ou copolymère biodégradable lipophile est choisi dans l'ensemble constitué par le poly(acide glycolique) (PGA), le copoly(acide lactique/acide glycolique) (PLGA), le poly(acide γ-glutamique), le poly(acide vinylique) (PVA), la polycaprolactone, les polyanhydrides, un poly(acide aminé), la polydioxanone, un polyhydroxybutyrate, les polyphosphazènes, un polyester-uréthane, le copoly(carboxyphénoxypropane/acide sébacique), un polyorthoester, et leurs combinaisons.

9. Procédé selon la revendication 6, dans lequel le polymère ou copolymère biodégradable lipophile est le copoly(acide lactique/acide glycolique) (PLGA).

10. Procédé selon la revendication 6, dans lequel le tensioactif est choisi dans l'ensemble constitué par un ester d'acide gras et de glycérol, un ester d'acide gras et de saccharose, un ester d'acide gras et de sorbitan, un ester d'acide gras et de propylèneglycol, les esters d'acide diacétyltartrique et de mono- et di-glycérides, le phosphate de sodium-aluminium, le polysorbate 20, le polysorbate 60, le polysorbate 65, le polysorbate 80, l'hydroxypropylcellulose, l'hydroxypropyl-méthylcellulose, les mono- et di-glycérides citratés, les mono- et di-glycérides tartratés, les mono- et di-glycérides lactatés, les mono- et di-glycérides éthoxylés, les dérivés mono- et di-glycéridiques de phosphate monosodique, les monoglycérides succinylatés, les esters de polyglycérol et d'acide gras, les esters de polyglycérol et d'acide ricinoléique inter-estérifié, le stéaryl-2-lactylate de calcium, les sels d'acides gras, le monopalmitate de sorbitan polyoxyéthyléné(20), le polysorbate 40, un monostéarate polyoxyéthyléné(20), le tristéarate de sorbitan polyoxyéthyléné(20), le Triton X-100, les polyéthylèneglycols 200-800, le laurylsulfate de sodium, les alcools éthoxylés, les alkylphénois éthoxylés, les alkylpolygiycosides, et leurs combinaisons.

11. Procédé selon la revendication 6, dans lequel le solvant organique est choisi dans l'ensemble constitué par le dichlorométhane, le chloroforme, l'acétate d'éthyle, le 1,4-dioxane, le diméthylformamide (DMF), le diméthylsulfoxyde (DMSO), le toluène, le tétrahydrofurane (THF), et leurs combinaisons.
